# EUROPEAN PATENT SPECIFICATION

(11) **EP 1 683 418 B1**
(45) Date of publication and mention of the grant of the patent: **13.11.2013**
(21) Application number: 06100303.4
(22) Date of filing: 12.01.2006
(51) Int. Cl.: A01N 43/40, A01P 1/00, A61K 8/49, A61K 8/33, A61K 8/41, A61Q 19/10, A01N 33/24

(54) **Compositions for hygienic hand disinfection and disinfectant handwashing**
Zusammensetzungen zum Desinfizieren und Waschen der Händen
Compositions pour l'hygiène et la désinfection des mains

(30) Priority: 19.01.2005 DE 102005002643
(43) Date of publication of application: 26.07.2006
(73) Proprietor: Air Liquide Santé (International), 75341 Paris Cedex 07 (FR); Schuelke & Mayr, 22851 Norderstedt (DE)
(72) Inventor: Beilfuss, Wolfgang, 22339 HAMBURG (DE); Behrends, Sabine, 25421 APPEN (DE); Goroncy-Bermes, Peter, 22145 HAMBURG (DE); Puchstein, Burghard, 20257 HAMBURG (DE)
(74) Representative: Conan, Philippe Claude

(56) References cited:
- EP-A- 1 468 700
- GB-A- 1 533 952
- US-A- 4 542 125

## Description

The present invention relates to disinfectant compositions which are preferably employed for hygienic hand disinfection and disinfectant handwashing.

The aim of hand disinfection, hand decontamination and skin antisepsis is to prevent transmission of microorganisms and viruses or to suppress their unwanted introduction into endangered regions of the body or more sensitive regions. Compositions for hygienic hand disinfection and for disinfectant handwashing must satisfy certain efficacy requirements, some of which are defined in standards. Various methods are possible for treating the hands after contamination.

Hygienic hand disinfection complying with EN 1500 as rubbing method without addition of water causes the death or inactivation of the transient microorganisms on the hands, without any risk of microbes being disseminated in the surroundings and without any risk of recontamination of the hands by microorganisms possibly present in the water. Disinfectant handwashing complying with EN 1499 with a microbicidal composition using tap water is likewise directed against transient microorganisms without precluding their dissemination in the surroundings. It serves in particular to reduce microbes during the washing procedure, but cannot replace hand disinfection. In disinfectant handwashing, the composition is rubbed in undiluted and foamed with a little water, and the hands are cleaned and thoroughly rinsed with water.

Compositions for disinfectant handwashing and hygienic hand disinfection must be effective after acting for short times (e.g. 30 seconds or 1 minute). In the efficacy tests, it is important that there is a good effect (RF value > 3 orders of magnitude) after acting for these short times. For toxicological reasons it is additionally necessary for, besides adequate efficacy, in particular the compatibility with human skin to be ensured.

Commercially available compositions for disinfectant handwashing are usually alcohol- or surfactant-containing liquid soaps and wash lotions ready for use with further biocidal agents added. Known compositions comprise as biocidal agents for example short-chain alcohols and as excipients refatting agents, moisturizers and fragrances to improve the skin compatibility and acceptance. To enhance the efficacy and to achieve a residual effect intended to prevent the number of microbes on the hands increasing again, the known compositions often also comprise additional agents such as biguanides (e.g. chlorhexidine), quaternary ammonium compounds (e.g. benzalkonium chloride), phenol derivatives (e.g. ortho-phenylphenol) or carboxylic acids.

US-A 4 542 125 discloses combinations of octenidine dihydrochloride with (higher alkyl) (di-lower-alkyl)amine oxide as dip compositions for disinfecting the teats of cows. US-A 4 420 484 and DE 27 08 331 A1 describe antimicrobial skin-cleansing compositions which comprise octenidine dihydrochloride and optionally amine oxide.

DE 102 05 883 A1 relates to an aqueous antiseptic based on bispyridiniumalkanes. Besides octenidine dihydrochloride, the antiseptic comprises a nonionic surfactant selected from alcohol polyalkoxylates, polysorbates and alkyl glycosides. The further presence of amine oxide is emphasized as disadvantageous. DE 196 47 692 A1 relates to an aqueous disinfectant solution which comprises octenidine dihydrochloride, alkyl alcohol, nonionic and/or cationic surfactant and skin-compatible α-hydroxy carboxylic acid. The presence of amine oxides is referred to as disadvantageous.

GB 1533952 discloses bispyridiniumalkanes as antimicrobials in soaps and pre-operative skin treatments.

EP 1468700 discloses Sensiva™ SC 50 having intrinsically antimicrobial properties, a good compatibility with other agents and materials and wetting properties.

In addition, an aqueous composition which includes octenidine dihydrochloride, cocamidopropylamine oxide, glyceryl cocoate, glycerol, hydroxyethylcellulose, lactic acid and allantoin is known as commercial product.

The known compositions for disinfectant handwashing have a number of disadvantages. Thus, some products show an efficacy which is not always satisfactory, or the desired efficacy is achieved only after acting for a lengthy time. Some compositions additionally have insufficient skin compatibility. Thus, compositions based on chlorhexidine are reported to be prone to skin incompatibility, it being suggested for example that there is partial release of chloroaniline.

An additional factor is that agents with organically bound halogen such as chlorhexidine have only conditional environmental compatibility. Chlorhexidine is moreover sufficiently effective only with a comparatively high concentration of the agent (e.g. 2% by weight) in wash products and may lead to discolorations on contaminated surfaces. Poly(hexamethylenebiguanide) hydrochloride is a polymeric biguanide salt whose structure is not exactly defined. For this reason, no medicinal product with this agent yet has marketing authorization in Germany. The polymeric biguanide may be employed only in a concentration of up to 0.3% by weight in cosmetic compositions.

Hence there is a need for compositions with improved efficacy and, at the same time, good skin compatibility.

It has now surprisingly been found that the problems of the prior art can be overcome by a composition according to Claim 1. The compositions according to the invention have an excellent effect with, at the same time, good skin compatibility and stability. They show a distinctly improved efficacy compared with known compositions. Moreover, synergistic increases in effect are observed in some cases. The possible inhibition of the efficacy of bispyridiniumalkanes, which is observed with numerous surfactants, does not occur with amine oxides.

The compositions can be employed as products for personal hygiene and as medical wash products, high-value, soap-free wash products for all hand, skin and body washing and as bath additive, and for disinfection or decontamination of animate (e.g. skin, hands, mucous membrane, wounds) and inanimate surfaces (e.g. apparatuses, instruments, endoscopes).

### a) Glycerol monoalkyl ethers

According to the invention, the content of 1-(2-Ethylhexyl)-glycerol ether (Sensiva^{®} SC 50) is generally in the range from 0.05 to 5% by weight, preferably 0.1 to 3% by weight, more preferably 0.2 to 2% by weight, such as 0.5 to 1% by weight.

### b) Bispyridiniumalkane

The bispyridiniumalkane employed according to the invention is N,N'-(1,10-decanediyldi-1[4H]-pyridinyl-4-ylidene)bis(1-octanamine) dihydrochloride (octenidine dihydrochloride). The content of octenidine dihydrochloride is in the range from 0.01 to 2% by weight, more preferably 0.05 to 1.5% by weight, in particular 0.1 to 1% by weight, such as 0.2 to 0.5% by weight.

### c) Amine oxide

The amine oxide is cocamidopropylamine oxide, corresponding to the general formula

R¹R²R³N-O

Wherein R³ is R ⁴CONH(CH₂)ₙ where R⁴CO is the acyl radical derived from the fatty acids of coconut oil, n = 3, and R¹ and R² are methyl. In a preferred embodiment, the content of cocamidopropylamine oxide is in the range from 1 to 20% by weight, more preferably 2 to 15% by weight, in particular 6 to 13% by weight, such as 8 to 12% by weight, for example about 10% by weight.

Preference is moreover given to compositions which additionally include from 1 to 5% by weight, preferably 2 to 3% by weight, refatting agent, such as polyol fatty acid ester, e.g. glycerol ester, preferably glycerol cocoate, isopropyl myristate, isopropyl palmitate, and triglycerides.

Compositions which are additionally preferred are those additionally including from 0.05 to 0.5% by weight, more preferably 0.1 to 0.3% by weight, of skincare additive such as allantoin, glycerol or sodium gluconate.

A composition of the invention may additionally comprise from 0.1 to 5% by weight, preferably 0.5 to 2% by weight, of thickener, especially hydroxyethylcellulose, hydroxymethylcellulose and hydroxypropylcellulose.

To adjust the pH of the composition of the invention it is additionally possible for from 0.1 to 4% by weight, preferably 0.4 to 2% by weight, more preferably 0.6 to 1% by weight carboxylic acid to be present in the composition of the invention, such as hydroxy carboxylic acid, for example lactic acid, succinic acid, maleic acid, tartaric acid, citric acid or mandelic acid, with particular preference for lactic acid.

The composition of the invention is preferably in the form of an aqueous composition and comprises from 10 to 99% by weight, more preferably 30 to 98% by weight, in particular 50 to 95% by weight, such as 60 to 90% by weight, 65 to 88% by weight, 70 to 86% by weight or 80 to 85% by weight, based on the composition.

A particularly preferred composition includes:
a) 0.5 to 1% by weight, such as about 0.75% by weight, 1-(2-ethylhexyl)glycerol ether,
b) 0.2 to 0.5% by weight, such as about 0.3% by weight, octenidine dihydrochloride and
c) 8 to 12% by weight, such as about 10.0% by weight, cocamidopropylamine oxide,
and where appropriate
d) 0.8 to 1.5% by weight, such as about 1.1% by weight, glycerol,
e) 2 to 3% by weight, such as about 2.5% by weight, glycerol cocoate,
f) 0.1 to 0.3% by weight, such as about 0.2% by weight, allantoin,
g) 0.5 to 2% by weight, such as about 1.0% by weight, hydroxyethylcellulose,
h) 0.6 to 1% by weight, such as about 0.8%, lactic acid and/or
i) 80 to 85% by weight, such as about 83.15% by weight, water.

The composition may additionally comprise functional additives such as colorants, perfume, buffers, electrolytes and moisturizing factors. The preferred pH of the compositions is from 4 to 8, preferably 4 to 6.

The invention further relates to the use of the compositions for hygienic hand disinfection or for disinfectant handwashing. The compositions according to the invention are suitable for hygienic and surgical hand disinfection, hygienic handwashing, hand decontamination, skin decontamination, mucous membrane decontamination, personal hygiene washing lotion, as antimicrobial washing lotion, for (whole) body washing and care in connection with MRSA (methicillin-resistant Staphylococcus aureus) and furthermore for disinfectant handwashing, for hygienic catheter care in patients, as handwashing product such as, for example, as antimicrobial soap, handwashing gel or handwashing lotion. They can be employed advantageously in all sectors with enhanced hygiene requirements in the medical and nonmedical sector, e.g. hospitals, medical practices, old people's and nursing homes, and in the food products and kitchen sectors.

Compositions according to the invention may also be formulated as alcoholic hand disinfectants, gel compositions, ointment compositions and antimicrobial coatings.

It was surprising that an effect is achieved after only a short time with compositions according to the invention containing glycerol monoalkyl ether for hand disinfection. Although glycerol monoalkyl ethers are known, for example from DE 42 40 674 C1, to act as deodorant agents, disinfection is immaterial for deodorants. For this reason, known deodorant agents merely have antimicrobial activity, whereas hand disinfectants must have microbicidal activity. An additional factor is that glycerol monoalkyl ethers on their own have virtually no microbicidal effect, and accordingly are unsuitable as (sole) agents for hand disinfection. It was therefore surprising that glycerol monoalkyl ethers contribute in the compositions according to the invention to the hand-disinfectant effect.

In addition, deodorant compositions which are intended by their nature to remain on the skin for a lengthy period are preferably free of surfactants. Thus, compatibility between deodorant agents and surfactants is immaterial in deodorant compositions. By contrast, it was surprising that compositions according to the invention can be formulated with up to 20% by weight amine oxide without the efficacy of the bispyridiniumalkanes and glycerol monoalkyl ethers being impaired.

The compositions according to the invention additionally have the following advantages:
- Good compatibility in combination with good antimicrobial efficacy and excellent short-term effect (acting for a time of, for example, 30 or 60 seconds), and very good effect on Gram-negative bacteria.
- Good efficacy for viruses (enveloped and non-enveloped) and multidrug-resistant microorganisms such as MRSA.
- No need to add a further preservative because they are self-preservative.
- The solubility in water and water-based compositions of glycerol monoalkyl ethers (such as 1-(2-ethylhexyl)glycerol ether) is only limited (solubility in water 0.1% by weight). The presence of bispyridiniumalkane in the compositions according to the invention leads to a significantly improved solubility in water of the glycerol monoalkyl ether.

The advantages of the invention are evident in particular from the following examples.

### Examples

The following compositions were formulated. All data are in % by weight.

| | A | B (comparative) |
|---|---|---|
| Polybiguanide | - | - |
| Sensiva® SC 50 | 0.75 | - |
| Octenidine dihydrochloride | 0.30 | 0.30 |
| Fatty acid amidopropyldimethylamine oxide | 10.00 | 10.00 |
| Glycerol | 1.10 | 1.10 |
| Allantoin | 0.20 | 0.20 |
| Glycerol cocoate | 2.50 | 2.50 |
| Hydroxyethylcellulose | 1.00 | 1.00 |
| Lactic acid | 0.81 | 0.81 |
| Alkyl polyglycoside | - | - |
| PEG 150 distearate | - | - |
| Deionized water | ad 100 | ad 100 |

| | | |
|---|---|---|
| ¹ Amount not known. | | |

### Method for determining the efficacy of the compositions

The efficacy of the compositions of the invention was tested in the quantitative suspension test. Compare standard methods of the DGHM for testing chemical disinfection methods, J. Gebel, H.-P. Werner, A. Kirsch-Altena, K. Bansemir, mhp Verlag GmbH, Wiesbaden, Germany, Method 9.1 (date 1 September 2001) (quantitative suspension test with bacteria (apart from mycobacteria and fungi).

| S. aureus | A | B (comparative) |
|---|---|---|
| 15" | 1.83 | - |
| 30" | 3.81 | 0 |
| 1' | 4.50 | 2.38 |
| 3' | not determined | 3.52 |
| 5' | not determined | 4.28 |

| E. hirae | A | B (comparative) |
|---|---|---|
| 15" | 0 | not determined |
| 30" | 3.03 | 0 |
| 1' | 3.94 | 3.61 |

## Claims

1. Disinfectant composition which includes
a) 1-(2-ethylhexyl)glycerol ether,
b) octenidine dihydrochloride and
c) cocamidopropylamine oxide.

2. Composition according to Claim 1, **characterized in that** it includes from 0.05 to 5% by weight of 1-(2-ethylhexyl)glycerol ether.

3. Composition according to any of the preceding claims, **characterized in that** it includes from 0.01 to 2% by weight of octenidine dihydrochloride.

4. Composition according to any of the preceding claims, **characterized in that** it includes from 6 to 13% by weight cocamidopropylamine oxide..

5. Composition according to any of the preceding claims, **characterized in that** it additionally includes from 0.5 to 5% by weight glycerol.

6. Composition according to any of the preceding claims, **characterized in that** it additionally includes from 1 to 5% by weight glycerol cocoate.

7. Composition according to any of the preceding claims, **characterized in that** it additionally includes from 0.05 to 0.5% by weight allantoin.

8. Composition according to any of the preceding claims, **characterized in that** it additionally includes from 0.1 to 5% by weight hydroxyethylcellulose.

9. Composition according to any of the preceding claims, **characterized in that** it additionally includes from 0.1 to 4% by weight hydroxy carboxylic acid chosen from lactic acid, succinic acid, maleic acid, tartaric acid, citric acid or mandelic acid.

10. Composition according to any of the preceding claims, **characterized in that** it additionally includes from 60 to 90% by weight water.

11. Composition according to any of the preceding claims, **characterized in that** it includes
a) 0.5 to 1% by weight 1-(2-ethylhexyl)glycerol ether,
b) 0.2 to 0.5% by weight octenidine dihydrochloride and
c) 8 to 12% by weight cocamidopropylamine oxide, and where appropriate
d) 0.8 to 1.5% by weight glycerol,
e) 2 to 3% by weight glycerol cocoate,
f) 0.1 to 0.3% by weight allantoin,
g) 0.5 to 2% by weight hydroxyethylcellulose,
h) 0.6 to 1% by weight lactic acid and/or
i) 80 to 85% by weight water.

12. Non-therapeutic use of a composition according to any of the preceding claims,for hygienic hand disinfection or for disinfectant handwashing.

## Patentansprüche

1. Desinfektionsmittelzusammensetzung, die Folgendes enthält:
a) 1-(2-Ethylhexyl)glycerolether,
b) Octenidindihydrochlorid und
c) Cocamidopropylaminoxid.

2. Zusammensetzung nach Anspruch 1, **dadurch gekennzeichnet, dass** sie 0,05 bis 5 Gew.-% 1-(2-Ethylhexyl)glycerolether enthält.

3. Zusammensetzung nach einem der vorherigen Ansprüche, **dadurch gekennzeichnet, dass** sie 0,01 bis 2 Gew.-% Octenidindihydrochlorid enthält.

4. Zusammensetzung nach einem der vorherigen Ansprüche, **dadurch gekennzeichnet, dass** sie 6 bis 13 Gew.-% Cocamidopropylaminoxid enthält.

5. Zusammensetzung nach einem der vorherigen Ansprüche, **dadurch gekennzeichnet, dass** sie zusätzlich 0,5 bis 5 Gew.-% Glycerol enthält.

6. Zusammensetzung nach einem der vorherigen Ansprüche, **dadurch gekennzeichnet, dass** sie zusätzlich 1 bis 5 Gew.-% Glycerolcocoat enthält.

7. Zusammensetzung nach einem der vorherigen Ansprüche, **dadurch gekennzeichnet, dass** sie zusätzlich 0,05 bis 0,5 Gew.-% Allantoin enthält.

8. Zusammensetzung nach einem der vorherigen Ansprüche, **dadurch gekennzeichnet, dass** sie zusätzlich 0,1 bis 5 Gew.-% Hydroxyethylcellulose enthält.

9. Zusammensetzung nach einem der vorherigen Ansprüche, **dadurch gekennzeichnet, dass** sie zusätzlich 0,1 bis 4 Gew.-% Hydroxycarbonsäure enthält, die aus Milchsäure, Bernsteinsäure, Maleinsäure, Weinsäure, Zitronensäure oder Mandelsäure ausgewählt ist.

10. Zusammensetzung nach einem der vorherigen Ansprüche, **dadurch gekennzeichnet, dass** sie zusätzlich 60 bis 90 Gew.-% Wasser enthält.

11. Zusammensetzung nach einem der vorherigen Ansprüche, **dadurch gekennzeichnet, dass** sie Folgendes enthält:
a) 0,5 bis 1 Gew.-% 1-(2-Ethylhexyl)glycerolether,
b) 0,2 bis 0,5 Gew.-% Octenidindihydrochlorid und
c) 8 bis 12 Gew.-% Cocamidopropylaminoxid, und gegebenenfalls
d) 0,8 bis 1,5 Gew.-% Glycerol,
e) 2 bis 3 Gew.-% Glycerolcocoat,
f) 0,1 bis 0,3 Gew.-% Allantoin,
g) 0,5 bis 2 Gew.-% Hydroxyethylcellulose,
h) 0,6 bis 1 Gew.-% Milchsäure und/oder
i) 80 bis 85 Gew.-% Wasser.

12. Nichttherapeutische Verwendung einer Zusammensetzung nach einem der vorherigen Ansprüche zur hygienischen Händedesinfektion oder zum desinfizierenden Händewaschen.

## Revendications

1. Composition désinfectante qui comprend
a) de l'éther de 1-(2-éthylhexyl)glycérol,
b) du dichlorhydrate d'octénidine et
c) de l'oxyde de cocamidopropylamine.

2. Composition selon la revendication 1, **caractérisée en ce qu'**elle comprend de 0,05 à 5 % en poids d'éther de 1-(2-éthylhexyl)glycérol.

3. Composition selon l'une quelconque des revendications précédentes, **caractérisée en ce qu'**elle comprend de 0,01 à 2 % en poids de dichlorhydrate d'octénidine.

4. Composition selon l'une quelconque des revendications précédentes, **caractérisée en ce qu'**elle comprend de 6 à 13 % en poids d'oxyde de cocamidopropylamine.

5. Composition selon l'une quelconque des revendications précédentes, **caractérisée en ce qu'**elle comprend en outre de 0,5 à 5 % en poids de glycérol.

6. Composition selon l'une quelconque des revendications précédentes, **caractérisée en ce qu'**elle comprend en outre de 1 à 5 % en poids de cocoate de glycérol.

7. Composition selon l'une quelcnque des revendications précédentes, **caractérisée en ce qu'**elle comprend en outre de 0,05 à 0,5 % en poids d'allantoïne.

8. Composition selon l'une quelconque des revendications précédentes, **caractérisée en ce qu'**elle comprend en outre de 0,1 à 5 % en poids d'hydroxyéthylcellulose.

9. Composition selon l'une quelconque des revendications précédentes, **caractérisée en ce qu'**elle comprend en outre de 0,1 à 4 % en poids d'acide hydroxycarboxylique choisi parmi l'acide lactique, l'acide succinique, l'acide maléique, l'acide tartrique, l'acide citrique ou l'acide mandélique.

10. Composition selon l'une quelconque des revendications précédentes, **caractérisée en ce qu'**elle comprend en outre de 60 à 90 % en poids d'eau.

11. Composition selon l'une quelconque des revendications précédentes, **caractérisée en ce qu'**elle comprend en outre
a) de 0,5 à 1 % en poids d'éther de 1-(2-éthylhexyl)glycérol,
b) de 0,2 à 0,5 % en poids de dichlorhydrate d'octénidine et
c) de 8 à 12 % en poids d'oxyde de cocamidopropylamine, et si approprié,
d) de 0,8 % à 1,5 % en poids de glycérol,
e) de 2 à 3 % en poids de cocoate de glycérol,
f) de 0,1 à 0,3 % en poids d'allantoïne,
g) de 0,5 à 2 % en poids d'hydroxyéthylcellulose,
h) de 0,6 à 1 % en poids d'acide lactique et/ou
i) de 80 à 85 % en poids d'eau.

12. Utilisation non thérapeutique d'une composition selon l'une quelconque des revendications précédentes, destinée à la désinfection hygiénique des mains ou à un lavage désinfectant des mains.
